# EUROPEAN PATENT SPECIFICATION

(11) **EP 3 769 986 B1**
(45) Date of publication and mention of the grant of the patent: **02.12.2026**
(21) Application number: 19188111.9
(22) Date of filing: 24.07.2019
(51) Int. Cl.: B60H 3/06, A61L 9/20, B01D 53/86

(54) **METHOD OF DECREASING THE CONCENTRATION OF NITROGEN OXIDES AND SULFUR OXIDES IN AIR**
VERFAHREN ZUR VERRINGERUNG DER KONZENTRATION VON STICKOXIDEN UND SCHWEFELOXIDEN IN DER LUFT
PROCÉDÉ DE DIMINUTION DE LA CONCENTRATION D'OXYDES D'AZOTE ET D'OXYDES DE SOUFRE DANS L'AIR

(43) Date of publication of application: 27.01.2021
(73) Proprietor: Bayerische Motoren Werke Aktiengesellschaft, 80809 München (DE)
(72) Inventor: Gentschev, Ann-Christin, 80637 München (DE); Iliffe-Moon, Etienne, Menlo Park, 94025 (US)

(56) References cited:
- EP-A1- 3 431 164
- EP-A2- 2 383 130
- CN-A- 106 739 984
- CN-A- 108 240 687
- CN-A- 108 883 205
- DE-A1- 102004 034 432
- JP-A- 2000 343 936
- US-A1- 2018 319 256

## Description

The present invention relates to a method of decreasing the concentration of nitrogen oxides and sulfur oxides in air polluted with said oxides. The invention further relates to a passenger compartment of a vehicle comprising a device configured to perform the method.

Exhaust emissions from vehicles powered by a combustion engine, mainly diesel cars, may pollute air with nitrogen oxides (NOₓ) and sulfur oxides (SOₓ). Further emissions generated by combustion are typically water and volatile organic compounds (VOC).

NOₓ and SOₓ are potentially dangerous to health of a human when inhaled. Moreover, NOₓ and SOₓ may be responsible for the formation of toxic ground-level ozone which e.g. frequently may be identified because of its characteristic smell. Due to growing environmental impacts, the concentration of such pollutants in air should be kept as low as possible.

In particular during rush hours, passengers of a vehicle may be exposed to increased NOₓ and SOₓ concentrations when said air comprising NOₓ and SOₓ has access to said vehicles.

EP 2 383 130 A3 and JP 2000 343936 A disclose methods of converting or destroying pollutants contained in air by a photocatalytic reaction.

DE 10 2004 034 432 discloses a purification method of air using a catalytic agent.

CN 108 883 205 A discloses a photocatalytic sterilization device and a refrigerator including the photocatalytic sterilization device.

EP 3 431 164 A1 discloses an air purification unit. The air purification unit has at least one region permeated with activated carbon and a photocatalytically active region.

CN 106 739 984 A discloses a photocatalyst inverter which comprises an inverter body and a circuit board.

CN 108 240 687 A discloses a deodorizing method and a deodorizing apparatus for realizing the method mentioned above.

US 2018/319256 A1 discloses a ventilation system for a mobile structure, in particular a motor vehicle.

It would be desirable to implement a suitable system in a vehicle configured to prevent the passengers of said vehicles to inhale nitrogen oxides (NOₓ) and sulfur oxides (SOₓ) or at least to decrease the concentration of said oxides since said oxides are more critical in a closed environment such as a passenger compartment of a vehicle due to the surrounding traffic.

This object could be achieved with a method of decreasing the concentration of NOₓ or SOₓ or NOₓ and SOₓ in air in the interior of a passenger compartment of a vehicle, said air comprising said oxides, as defined in claim 1.

Without being bound by theory, the inventors assume that the chemical basis of the new method is the formation of hydroxyl radicals possibly stemming from water, wherein said photocatalytic coating catalyzes the formation of said radicals. Such radicals may easily react with volatile organic compounds (VOC) upon forming water and hydrocarbon radicals. Such hydrocarbon radicals may in turn react with oxygen to form hydrocarbon peroxy radicals. Such hydrocarbon peroxy radicals as well as said hydroxyl radicals may destroy NOₓ according to reactions that are basically known in the art, and associated similar mechanisms concerning the destruction of SOₓ.

Accordingly, the method according to the invention is based on a chemical reaction between NOₓ or SOₓ, water, VOCs, and oxygen, wherein said oxides are destroyed.

Since the radical formation takes place at the surface of the photocatalytic coating and, according to known mechanisms in the field of photocatalysis, the radicals are bound to said surface of the coating, advantageously none of said radicals may pollute the air inside the compartment.

Moreover, due to the bonding of said radicals to said surface of the coating, no unpleasant smell due to free radicals will result.

Preferably, said water and volatile organic compounds used in step (A) also stem from said air being guided to said passenger compartment, i.e. said water and volatile organic compounds are also comprised in said air.

The term "decreasing the concentration of" as used herein may be synonymously used with a term such as "minimizing the concentration of".

The terms "decreasing the concentration of" or "minimizing the concentration of" relate to a comparison with air being outside said passenger compartment of a vehicle.

In one embodiment, the term "decreasing the concentration of" may be synonymously used with the term "destroying".

Accordingly, in one embodiment, the invention relates to a method of destroying NOₓ and SOₓ in air in the interior of a passenger compartment of a vehicle, said air comprising said oxide or oxides, the method comprising steps (A) and (B):
(A) guiding air from the environment of said compartment to said compartment, said air comprising NOₓ and SOₓ in presence of water and volatile organic compounds; and
(B) contacting said air with a photocatalytic coating in the presence of photons configured to activate said photocatalytic coating in order to destroy NOₓ and SOₓ before said air reaches the interior of the vehicle;

wherein the photocatalytic coating used in step (B) is disposed on fibers forming a porous lattice configured to be permeable for said air;
wherein the photocatalytic coating comprises photocatalytic particles,
wherein the photocatalytic coating comprises photocatalytic particles selected from TiO₂ doped with iron.

The term "water" as used herein encompasses water in the gaseous phase, i.e. vaporized water. Said water can stem from water emitted by combustion engines or from natural air humidity or from artificial humidification.

The term "volatile organic compound (VOC)" as used herein encompasses any chemical compound based on carbon which is released by natural sources or by human activity into the air, e.g. by use of solvents, dyes and lacquers, storage of fuels e.g. at filling stations and emissions from combustion processes using fuel.

The term "photocatalytic coating" as used herein encompasses any coating which can be activated by photons configured to activate this coating. The activation mechanisms are known in the art.

Preferably, said photocatalytic coating is made from photocatalytic metal oxides.

In one embodiment, the photocatalytic coating comprises photocatalytic particles.

The term "photons configured to activate said photocatalytic coating or particles" as used herein means that the photons have a wavelength corresponding to at least a band gap energy of the photocatalytic coating or the photocatalytic particles. Accordingly, the photocatalytic coating or the photocatalytic coating comprising photocatalytic particles typically have semiconductor properties.

In a preferred embodiment of the method, said contacting in step (B) is performed in a device comprising said photocatalytic coating.

Preferably, said air used in step (A) flows through said device.

In one embodiment, the air flow is caused by suction or blowing.

Said device may be arranged at or in any location of the passenger compartment, provided the location of the compartment allows performing the method.

Preferably, the photocatalytic metal oxides used for the coating of step (B) or used for photocatalytic particles are selected from the group consisting of TiO₂, Bi₂O₃, WO₃, ZnO, FeO, SnO, and SiO₂, and two or more thereof.

Said metal oxides may also be doped with dopants in order to adapt the band gap energy of the photocatalytic particle to a determined wavelength of the photons.

According to the invention, TiO₂ doped with iron is used.

In one embodiment, said photocatalytic particles are present in the form of nanoparticles.

The term "nanoparticles" as used herein encompasses particles having a diameter in the range from 5 to 100 nm.

The photocatalytic coating is disposed on a porous support configured to be permeable for said air.

The term "porous support" as used herein encompasses any material that is suitable to form pores, e.g., but not limited to, porous glasses, porous carbon, porous oxides, porous ceramics or porous fibers.

The porous support may be present in form of a mold.

The support comprises fibers such as fibers based on natural or synthetic fibers.

In another embodiment, the porous support consists of fibers such as fibers based on natural or synthetic fibers.

Preferably, the fibers are composed of cotton, wool, polymers, metals, metal oxides or carbon fibers and may be woven or non-woven to form a porous lattice configured to be permeable for said air. In this embodiment, the fibers function as the surface upon which the photocatalytic particles in form of a photocatalytic coating are attached.

Preferably, the fibers are configured to act as filter or screen.

A suitable filter system or screen is e.g. known from US 2019/0046680 A1 (Molekule Inc.). This filter system may be adapted to the method described herein, if necessary. The filter system of this reference is used to decontaminate a fluid.

In a further embodiment, the method further comprises in step (B) activating said photocatalytic coating by means of photons emitted by a suitable photon source.

In a preferred embodiment, the photon source is configured to emit ultraviolet (UV) light. Accordingly, in one embodiment, the photon source comprises an emitting source such as a lamp or a LED or several lamps or LEDs.

The term "UV light" as used herein encompasses a wavelength range of from 100 nm to 400 nm. Specifically mentioned have to be UV-A radiation in a range of from 315 to 400 nm, UV-B radiation in a range of from 280 to 315 nm and UV-C radiation in a range of from 100 to 280 nm.

The method according to the invention may be further combined with known methods of improving the air quality in compartments configured to accommodate a human, e.g. filtering air in order to decrease or minimize the concentration of dust, allergens or germs or two or three thereof in the air.

The invention further relates to a passenger compartment of a vehicle, the compartment comprising a device comprising a photocatalytic coating and a source configured to emit photons, the device being configured to perform the method according to any of claims 1-5.

## Claims

1. A method of decreasing the concentration of NOₓ and SOₓ in air in the interior of a passenger compartment of a vehicle, said air comprising said oxide or oxides, the method comprising steps (A) and (B):
(A) guiding air from the environment of said compartment to said compartment, said air comprising NOₓ and SOₓ in presence of water and volatile organic compounds; and
(B) contacting said air with a photocatalytic coating in the presence of photons configured to activate said photocatalytic coating in order to destroy NOₓ and SOₓ before said air reaches the interior of the compartment;
wherein the photocatalytic coating used in step (B) is disposed on fibers forming a porous lattice configured to be permeable for said air;
wherein the photocatalytic coating comprises photocatalytic particles,
**characterized in that** the photocatalytic coating comprises photocatalytic particles selected from TiO₂ doped with iron.

2. The method of claim 1, wherein said contacting in step (B) is performed in a device comprising said photocatalytic coating, wherein said air used in step (A) flows through said device.

3. The method of any of the preceding claims, wherein in step (B) the photons are generated in a photon source.

4. The method of claim 3, wherein the photon source is configured to emit UV light.

5. The method according to any of the preceding claims, wherein the method is combined with a method of decreasing the concentration of dust, allergens or germs or two or three thereof in the air used in step (A).

6. A passenger compartment of a vehicle, the compartment comprising a device comprising a photocatalytic coating and a source configured to emit photons, the device being configured to perform the method as defined in any one of claims 1 to 5.

## Patentansprüche

1. Verfahren zur Verringerung der Konzentration von NOx und SOx in Luft im Inneren eines Fahrgastraums eines Fahrzeugs, wobei die Luft das Oxid oder die Oxide umfasst, wobei das Verfahren die Schritte (A) und (B) umfasst:
(A) Leiten von Luft aus der Umgebung des Fahrgastraums zu dem Fahrgastraum, wobei die Luft NOx und SOx in Gegenwart von Wasser und flüchtigen organischen Verbindungen umfasst; und
(B) In-Kontakt-Bringen der Luft mit einer photokatalytischen Beschichtung in Gegenwart von Photonen, die dazu ausgebildet sind, die photokatalytische Beschichtung zu aktivieren, um NOx und SOx zu zerstören, bevor die Luft das Innere des Fahrgastraums erreicht;
wobei die in Schritt (B) verwendete photokatalytische Beschichtung auf Fasern angeordnet ist, die ein poröses Gitter bilden, das dazu ausgebildet ist, für die Luft durchlässig zu sein;
wobei die photokatalytische Beschichtung photokatalytische Partikel umfasst,
**dadurch gekennzeichnet, dass** die photokatalytische Beschichtung photokatalytische Partikel umfasst, die aus mit Eisen dotiertem TiO2 ausgewählt sind.

2. Verfahren nach Anspruch 1, wobei das In-Kontakt-Bringen in Schritt (B) in einer Vorrichtung durchgeführt wird, die die photokatalytische Beschichtung umfasst, wobei die in Schritt (A) verwendete Luft durch die Vorrichtung strömt.

3. Verfahren nach einem der vorhergehenden Ansprüche, wobei in Schritt (B) die Photonen in einer Photonenquelle erzeugt werden.

4. Verfahren nach Anspruch 3, wobei die Photonenquelle dazu ausgebildet ist, UV-Licht zu emittieren.

5. Verfahren nach einem der vorhergehenden Ansprüche, wobei das Verfahren mit einem Verfahren zur Verringerung der Konzentration von Staub, Allergenen oder Keimen oder zweien oder dreien davon in der in Schritt (A) verwendeten Luft kombiniert wird.

6. Fahrgastraum eines Fahrzeugs, wobei der Fahrgastraum eine Vorrichtung umfasst, die eine photokatalytische Beschichtung und eine Quelle, die dazu ausgebildet ist, Photonen zu emittieren, umfasst, wobei die Vorrichtung dazu ausgebildet ist, das Verfahren gemäß einem der Ansprüche 1 bis 5 durchzuführen.

## Revendications

1. Procédé de diminution de la concentration de NOx et de SOx dans l'air à l'intérieur d'un compartiment passagers d'un véhicule, ledit air comprenant ledit oxyde ou lesdits oxydes, le procédé comprenant les étapes (A) et (B) :
(A) le guidage de l'air depuis l'environnement dudit compartiment vers ledit compartiment, ledit air comprenant du NOx et du SOx en présence d'eau et de composés organiques volatils ; et
(B) la mise en contact dudit air avec un revêtement photocatalytique en présence de photons configurés pour activer ledit revêtement photocatalytique afin de détruire le NOx et le SOx avant que ledit air n'atteigne l'intérieur du compartiment ;
dans lequel le revêtement photocatalytique utilisé à l'étape (B) est disposé sur des fibres formant un réseau poreux configuré pour être perméable audit air ;
dans lequel le revêtement photocatalytique comprend des particules photocatalytiques,
**caractérisé en ce que** le revêtement photocatalytique comprend des particules photocatalytiques choisies parmi du TiO2 dopé au fer.

2. Procédé selon la revendication 1, dans lequel ladite mise en contact à l'étape (B) est réalisée dans un dispositif comprenant ledit revêtement photocatalytique, dans lequel ledit air utilisé à l'étape (A) s'écoule à travers ledit dispositif.

3. Procédé selon l'une quelconque des revendications précédentes, dans lequel à l'étape (B), les photons sont générés dans une source de photons.

4. Procédé selon la revendication 3, dans lequel la source de photons est configurée pour émettre une lumière UV.

5. Procédé selon l'une quelconque des revendications précédentes, dans lequel le procédé est combiné avec un procédé de diminution de la concentration de poussière, d'allergènes ou de germes ou de deux ou trois d'entre eux dans l'air utilisé à l'étape (A).

6. Compartiment passagers d'un véhicule, le compartiment comprenant un dispositif comprenant un revêtement photocatalytique et une source configurée pour émettre des photons, le dispositif étant configuré pour réaliser le procédé tel que défini dans l'une quelconque des revendications 1 à 5.
